# EUROPEAN PATENT APPLICATION

(11) **EP 4 768 899 A1**
(43) Date of publication of application: **01.07.2026**
(21) Application number: 25186222.3
(22) Date of filing: 30.06.2025
(51) Int. Cl.: G01N 23/046, G06F 13/10

(54) **DATA ACQUISITION METHOD AND SYSTEM FOR STATIC COMPUTED TOMOGRAPHY, AND CT DEVICE**

(30) Priority: 27.12.2024 CN 202411957703
(71) Applicant: NUCTECH COMPANY LIMITED, Beijing 100084 (CN); Tsinghua University, Haidian District, Beijing 100084 (CN)
(72) Inventor: CHEN, Zhiqiang, Beijing, 100084 (CN); ZHANG, Li, Beijing, 100084 (CN); FENG, Bo, Beijing, 100084 (CN); ZHENG, Xianguo, Beijing, 100084 (CN); LI, Yuanjing, Beijing, 100084 (CN); HUANG, Qingping, Beijing, 100084 (CN); LIU, Yanqing, Beijing, 100084 (CN); TIAN, Shenghao, Beijing, 100084 (CN); ZHOU, Haijian, Beijing, 100084 (CN)
(74) Representative: Isarpatent

(57) **Abstract**

The present disclosure provides a data acquisition method and system for static computed tomography, and a CT device. The data acquisition method includes: sending (S01) a scan control system instruction to a master controller; parsing (S02) the scan control system instruction to generate an internal control instruction; sending (S03) the internal control instruction to a plurality of acquisition controllers; and controlling (S04) a plurality of detectors to perform data acquisition according to the internal control instruction.

## Description

### TECHNICAL FIELD

The present disclosure relates to a field of radiation detection technology, and in particular to a data acquisition method and system for static computed tomography, and a CT device.

### BACKGROUND

Static computed tomography (CT) scanning devices are widely used in fields such as medicine, security inspection, and industry. Static CT scanning devices utilize a plurality of radiation sources that sequentially emit scanning rays to achieve an effect of rotational scanning of an object to be inspected. Typically, a static CT scanning device includes a plurality of scanning beam planes, each responsible for scanning from a different angle to enable multi-angle scanning coverage. In a static CT scanning device, each scanning beam plane functions as an independent imaging system, including an optical-mechanical system, one or more acquisition controllers, and a detector array.

In related art, all scanning beam planes share a single set of control signals, which are distributed via wiring to the acquisition controllers of each scanning beam plane. This results in a large number of cables, and the dispersed positions of the acquisition controllers lead to complex and difficult wiring of control signal cables. Since all acquisition controllers need to perform data acquisition synchronously, a scan control system needs to provide a beam plane synchronization signal. Furthermore, all acquisition controllers are connected to a CAN bus, and each acquisition controller functions as an independent CAN node. The scan control system needs to maintain CAN communication links with all acquisition controllers, which results in a large number of CAN nodes and increases a risk of system failures.

### SUMMARY

In view of this, embodiments of the present disclosure provide a data acquisition method and system for static computed tomography, and a CT device.

In an aspect of the present disclosure, a data acquisition method for static computed tomography is provided, including: sending a scan control system instruction to a master controller; parsing the scan control system instruction to generate an internal control instruction; sending the internal control instruction to a plurality of acquisition controllers; and controlling a plurality of detectors to perform data acquisition according to the internal control instruction.

According to an embodiment of the present disclosure, the sending a scan control system instruction to a master controller includes: sending the scan control system instruction to the master controller by a scan control system via a controller area network bus.

According to an embodiment of the present disclosure, the sending the internal control instruction to a plurality of acquisition controllers includes: sending the internal control instruction to the plurality of acquisition controllers by the master controller via a plurality of high-speed serial interfaces.

According to an embodiment of the present disclosure, the scan control system instruction includes an angle coded A-direction pulse signal, an angle coded Z-direction pulse signal, a belt coded A-direction pulse signal, and a belt coded Z-direction pulse signal.

According to an embodiment of the present disclosure, the parsing the scan control system instruction to generate an internal control instruction includes: generating a sampling control signal and a sampling time information according to the scan control system instruction; and encoding the sampling control signal and the sampling time information to generate the internal control instruction.

According to an embodiment of the present disclosure, the sampling time information includes angle code A, angle code Z, belt code A, and belt code Z.

According to an embodiment of the present disclosure, the method further includes: uploading acquired data to the corresponding plurality of acquisition controllers by the plurality of detectors; packetizing the acquired data and the sampling time information by the plurality of acquisition controllers to obtain a plurality of data packets; and sending the plurality of data packets to the master controller by the plurality of acquisition controllers.

According to an embodiment of the present disclosure, the method further includes: uploading the plurality of data packets to an acquisition server by the master controller, where a data transmission between the master controller and the acquisition server is performed via a multi-port 10-gigabit Ethernet or a high-speed serial computer expansion bus.

According to an embodiment of the present disclosure, the method further includes: after completing data acquisition, generating a feedback instruction or handshake instruction by the plurality of acquisition controllers; sending the feedback instruction or handshake instruction to the master controller via the high-speed serial interface; parsing the feedback instruction or handshake instruction by the master controller to generate a corresponding controller area network bus feedback instruction or handshake instruction; and sending the corresponding controller area network bus feedback instruction or handshake instruction to the scan control system by the master controller via the controller area network bus

In another aspect, a data acquisition system for static computed tomography is provided, including: a scan control system configured to send a scan control system instruction to a master controller; the master controller configured to parse the scan control system instruction to generate an internal control instruction; a plurality of high-speed serial interfaces configured to transmit the internal control instruction to a plurality of acquisition controllers; and a plurality of scanning imaging systems, wherein the scanning imaging system includes one or more acquisition controllers, and the acquisition controller is configured to control a detector to perform data acquisition according to the internal control instruction.

According to an embodiment of the present disclosure, the scanning imaging system further includes: an optical-mechanical system configured to emit scanning rays; and one or more detectors configured to receive scanning rays that have passed through an object to be scanned, and generate detection data based on the received scanning rays.

According to an embodiment of the present disclosure, the acquisition controller is further configured to: packetize acquired data and sampling time information to obtain a plurality of data packets; and send the plurality of data packets to the master controller via the plurality of high-speed serial interfaces.

According to an embodiment of the present disclosure, the system further includes: a controller area network bus configured for signal transmission between the scan control system and the master controller.

According to an embodiment of the present disclosure, the system further includes an acquisition server, where a data transmission between the acquisition server and the master controller is performed via a multi-port 10-gigabit Ethernet or a high-speed serial computer expansion bus.

In another aspect of the present disclosure, a CT device is provided, including the data acquisition system as described above.

The above one or more embodiments have the following beneficial effects:
(1) All acquisition controllers are not connected to the CAN bus. Instead, an instruction interaction with the scan control system is achieved through the master controller as a hub, thereby reducing the number of CAN nodes in the system.
(2) The control signals of the scan control system, including angle coded A-direction pulse signals, angle coded Z-direction pulse signals, belt coded A-direction pulse signals, belt coded Z-direction pulse signals, etc., are not directly provided to the acquisition controllers. Instead, they are directly connected to the master controller, thereby reducing the number of system control signals.
(3) The internal control instruction is transmitted to all acquisition controllers via high-speed serial optical fiber interfaces, thereby achieving synchronous sampling of all beam planes, and the scan control system does not need to provide beam plane synchronization signals.
(4) The data transmission between the scan control system, the plurality of scanning beam planes and the acquisition server is achieved through the master controller, thereby improving the stability and reliability of the system.

### BRIEF DESCRIPTION OF THE DRAWINGS

Through the following description of embodiments of the present disclosure with reference to the accompanying drawings, the above and other objectives, features and advantages of the present disclosure will become more apparent. In the accompanying drawings:
FIG. 1 shows a schematic structural diagram of a static CT scanning device according to some embodiments of the present disclosure;
FIG. 2 shows a schematic structural diagram of a static CT scanning device according to other embodiments of the present disclosure;
FIG. 3 shows a schematic structural diagram of a data acquisition system for static CT scanning according to the related art;
FIG. 4 shows a partial flowchart of a data acquisition method for static CT scanning according to an embodiment of the present disclosure;
FIG. 5 shows a schematic structural diagram of a data packet according to an embodiment of the present disclosure;
FIG. 6 shows a partial flowchart of a data acquisition method for static CT scanning according to an embodiment of the present disclosure;
FIG. 7 shows a schematic structural diagram of a data acquisition system for static CT scanning according to an embodiment of the present disclosure;
FIG. 8 shows a structural block diagram of a CT device according to an embodiment of the present disclosure; and
FIG. 9 schematically shows a block diagram of an electronic device suitable for implementing the functions of controlling a CT device and/or the functions of processing data of the CT device according to an embodiment of the present disclosure.

It should be noted that, for the sake of clarity, the size of the overall/local structure or overall/local region in the drawings used to describe the embodiments of the present disclosure may be enlarged or reduced, that is, these drawings are not drawn to actual scale.

### DETAILED DESCRIPTION OF EMBODIMENTS

The following will describe the embodiments of the present disclosure with reference to the accompanying drawings. However, it should be understood that these descriptions are merely exemplary and are not intended to limit the scope of the present disclosure. In the following detailed description, for the purpose of explanation, numerous specific details are set forth to provide a comprehensive understanding of the embodiments of the present disclosure. However, it is apparent that one or more embodiments may be implemented without these specific details. Additionally, descriptions of well-known structures and technologies are omitted in the following description to avoid unnecessarily confusing the concepts of the present disclosure.

The terms used herein are only for describing specific embodiments and are not intended to limit the present disclosure. Terms such as "include" and "comprise" used herein indicate the presence of the stated features, steps, operations, and/or components, but do not exclude the presence or addition of one or more other features, steps, operations or components.

All terms (including technical and scientific terms) used herein have the meanings commonly understood by those skilled in the art unless otherwise defined. It should be noted that the terms used herein should be interpreted to have meanings consistent with the context of this specification and should not be interpreted in an idealized or overly rigid manner.

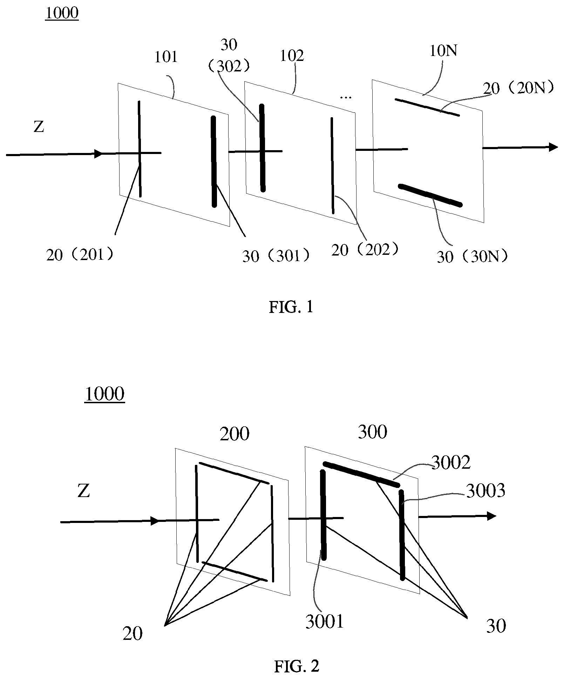

FIG. 1 shows a schematic structural diagram of a static CT scanning device according to some embodiments of the present disclosure. FIG. 2 shows a schematic structural diagram of a static CT scanning device according to other embodiments of the present disclosure.

A static CT scanning device typically includes one or more scanning beam planes. Each scanning beam plane may include an independent scanning imaging system. A plurality of scanning beam planes may be designed with a ring-shaped configuration or a non-ring-shaped configuration.

By way of example, referring to FIG. 1, a static CT scanning device 1000 with non-ring-shaped beam plane is provided. The static CT scanning device 1000 with non-ring-shaped beam plane may include a plurality of scanning beam planes arranged along a channel direction X, such as scanning beam plane 101, scanning beam plane 102, ..., scanning beam plane 10N, where N is a positive integer greater than or equal to 2. An object to be inspected may move along a channel direction Z and may be sequentially scanned at different angles by the scanning beam planes, thereby achieving overall multi-angle scanning coverage. For example, the channel direction Z may be a conveying direction of a conveyor belt. The object to be inspected may be placed on the conveyor belt and is sequentially scanned at different angles by each scanning beam plane as it moves along the channel direction Z with the conveyor belt.

It should be noted that in practical scenarios, a plurality of objects to be scanned may be placed on the conveyor belt simultaneously, each located at a different position on the conveyor belt. In other words, the static CT scanning device 1000 may scan and inspect a plurality of objects to be inspected simultaneously, thereby improving an inspection efficiency.

By way of example, a scanning beam plane may include a single scanning imaging system. Each scanning imaging system may include a detector array 20 and a multi-target array optical-mechanical system 30. The multi-target array optical-mechanical system 30 may also be referred to as an optical-mechanical system. The multi-target array optical-mechanical system 30 serves as a radiation source to emit scanning rays. The detector array 20 may receive the scanning rays transmitted through the object to be scanned, and generate detection data based on the received scanning rays. In the plurality of scanning imaging systems, each detector array 20 and its corresponding multi-target array optical-mechanical system 30 form a scanning beam plane. For example, a detector array 201 and a corresponding multi-target array optical-mechanical system 301 form a scanning beam plane 101, a detector array 202 and a corresponding multi-target array optical-mechanical system 302 form a scanning beam plane 102, and a detector array 20N and a corresponding multi-target array optical-mechanical system 30N form a scanning beam plane 10N.

As shown in FIG. 1, the scanning beam plane 101, the scanning beam plane 102 and the scanning beam plane 10N are distributed at different positions along the channel direction Z, and the object to be scanned may be sequentially transported by a conveying device (e.g., a conveyor belt) to regions of the scanning beam plane 101, the scanning beam plane 102 and the scanning beam plane 10N.

In an embodiment of the present disclosure, the respective multi-target array optical-mechanical systems 30 of the scanning beam plane 101, the scanning beam plane 102 and the scanning beam plane 10N have different ray paths, and thus may provide scans at different angles for the object to be scanned. The scanning beam plane 101, the scanning beam plane 102 and the scanning beam plane 10N may be perpendicular or inclined to the channel direction Z.

It should be noted that the number of scanning beam planes shown in FIG. 1 is for illustrative purposes only. Any number of scanning beam planes may be provided according to actual needs, which is not limited in the embodiments of the present disclosure.

By way of example, in a case where a static CT scanning device provides only three scanning beam planes, each scanning beam plane may provide a 120° scan for the object to be scanned, and scanning angles of the three 120° scans provided by the three scanning beam planes do not overlap with each other, thereby achieving a 360° scan of the object to be scanned.

In an embodiment of the present disclosure, the static CT scanning device 1000 may also be configured with ring-shaped beam planes. The static CT scanning device 1000 may include one or more ring-shaped beam planes.

By way of example, referring to FIG. 2, an embodiment of the present disclosure provides a static CT scanning device 1000 with a ring-shaped beam plane. The static CT scanning device 1000 may include a ring-shaped beam plane. In a scanning imaging system of a ring-shaped beam plane, a plurality of detector arrays are installed on the same geometric surface, and a plurality of multi-target array optical-mechanical systems are installed on the same geometric surface. The multi-target array optical-mechanical systems are deflected by a particular angle and sequentially emit rays according to a preset emission order, thus forming a ring-shaped scanning beam plane with the detector array. For example, a plurality of detector arrays 20 are located on the same physical plane to form a ring-shaped detector surface 200, and a plurality of multi-target array optical-mechanical systems 30 are located on the same physical plane to form an optical-mechanical surface 300. In each scanning imaging system, a plurality of multi-target array optical-mechanical systems 30 form a ring-shaped optical path. Each multi-target array optical-mechanical system is deflected by a particular angle, and all optical-mechanical systems point toward the corresponding ring-shaped detector surfaces 200. Each multi-target array optical-mechanical system 30 emits scanning rays to form a scanning beam plane.

By way of example, each multi-target array optical-mechanical system 30 may emit scanning rays in the form of a conical beam. The detector array 20 covered by the conical beam may collect the scanning rays transmitted through the object to be scanned, and the detector array 20 may generate detection data based on the received scanning rays. For example, each multi-target array optical-mechanical system 30 includes a plurality of targets, which emit scanning rays sequentially. For example, in at least one scanning imaging system, after a first target of the multi-target array optical-mechanical system 3001 emits scanning rays, a first target of the multi-target array optical-mechanical system 3002 emits scanning rays, followed by a first target of the multi-target array optical-mechanical system 3003. Subsequently, the second targets of the plurality of multi-target array optical-mechanical systems 3001, 3002 and 3003 may emit scanning rays in sequence, thereby achieving scanning of the object to be scanned passing through the scanning imaging system.

Whether the scanning beam planes are ring-shaped or non-ring-shaped, each beam plane may include an independent imaging system. To achieve control (e.g., timing control, angle control, etc.) over each scanning beam plane, each scanning beam plane includes a plurality of acquisition controllers in addition to the multi-target array optical-mechanical system and the detector array. The plurality of acquisition controllers may be used to respectively control a plurality of detectors in the detector array to perform data acquisition.

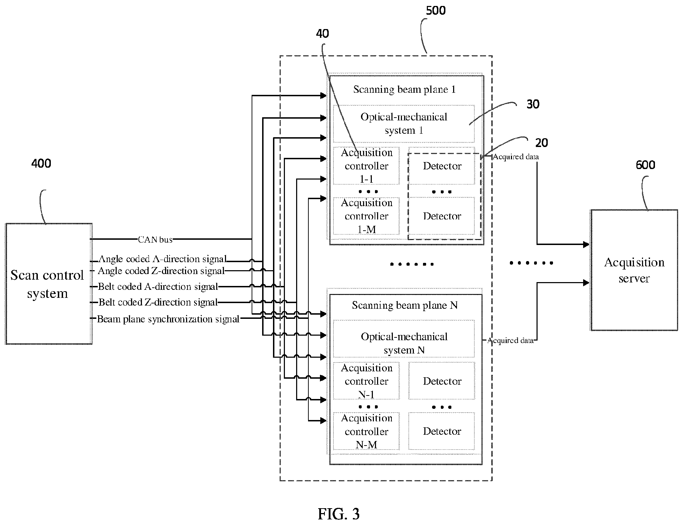

FIG. 3 shows a schematic structural diagram of a data acquisition system for static CT scanning according to the related art.

In related art, static CT scanning devices adopt a distributed data acquisition structure without a master control structure. For example, referring to FIG. 3, a static CT scanning device includes a scan control system 400, a plurality of scanning beam planes 500, and an acquisition server 600. The scan control system 400 may provide control signals to the plurality of scanning beam planes 500. The plurality of scanning beam planes 500 may scan at different angles under the control of the control signals. For example, each scanning beam plane 500 may include an optical-mechanical system 30 (i.e., multi-target array optical-mechanical system), a detector array 20, and a plurality of acquisition controllers 40. The plurality of acquisition controllers 40 may correspond to a plurality of detectors in the detector array 20 respectively. Each acquisition controller 40 may receive the control signals from the scan control system 400, so as to control the corresponding detector to perform data acquisition. The acquisition server 600 may receive acquired data from the plurality of scanning beam planes 500 and process the acquired data to form CT slice data.

The inventors have found through research that in a static CT scanning device of the related art, all beam planes share a common set of control signals, including CAN bus, angle coded pulse signals, belt coded pulse signals, beam plane synchronization pulse signals, etc. All control signals need to be wired to the acquisition controllers 40 of each beam plane, resulting in a large number of cables. Furthermore, the dispersed positions of the acquisition controllers 40 lead to complex and difficult wiring of control signal cables. Moreover, since all acquisition controllers 40 need to perform data acquisition synchronously, the scan control system 400 needs to provide a beam plane synchronization signal. Additionally, all acquisition controllers 40 are connected to the CAN bus, and each acquisition controller is an independent CAN node. As a result, the scan control system 400 needs to maintain CAN communication links with all acquisition controllers 40, resulting in a large number of CAN nodes and increasing the risk of system failures. Therefore, such a data acquisition system for static CT scanning based on a non-master control structure has high requirements for the quality of control signals. This leads to a complex system and presents significant risks to system stability.

To solve one or more of the above-mentioned technical problems, an embodiment of the present disclosure provides a data acquisition method and system for static CT scanning. By adding a master control structure, optimizing the connection relationships of various modules in the data acquisition system for static CT scanning, and optimizing the data acquisition method, it is possible to reduce the number of system control signals and the number of CAN nodes, thereby improving the stability and reliability of the system.

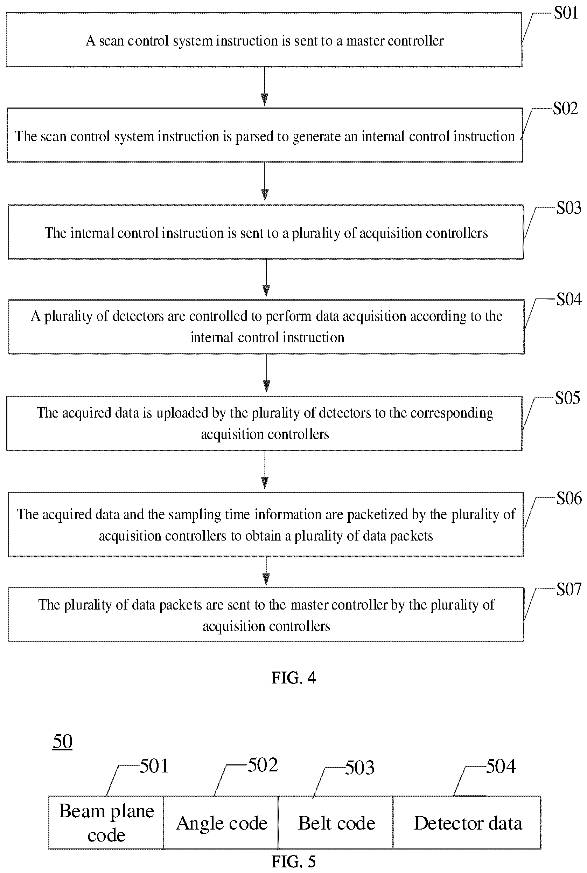

FIG. 4 shows a partial flowchart of a data acquisition method for static CT scanning according to an embodiment of the present disclosure.

By way of example, an embodiment of the present disclosure provides a data acquisition method for static CT scanning. Referring to FIG. 4, the data acquisition method may include step S01 to step S04.

In step S01, a scan control system instruction is sent to a master controller. For example, the scan control system instruction may include a CAN bus communication instruction. CAN bus communication is a distributed communication method that may transmit data between multiple devices. CAN bus communication may adopt an ID-based data frame transmission method to transmit data between different devices.

In step S02, the scan control system instruction is parsed to generate an internal control instruction. For example, the master controller may receive the CAN instruction from the scan control system, and then decode the CAN instruction to generate an internal control instruction. For example, the internal control instruction may include a serial communication instruction for transmitting data between different devices via a serial interface.

In step S03, the internal control instruction is sent to a plurality of acquisition controllers. For example, data may be transmitted between the master controller and the acquisition controller through serial interface communication in a point-to-point communication mode.

In step S04, a plurality of detectors are controlled to perform data acquisition according to the internal control instruction. For example, the acquisition controller may control a plurality of detectors in each scanning beam plane to sample according to a sampling control signal in the internal control instruction, thereby obtaining acquired data.

According to an embodiment of the present disclosure, sending the scan control system instruction to the master controller in step S01 may include: sending the scan control system instruction to the master controller by the scan control system via a controller area network bus (referred to as CAN bus). The scan system control instruction may be used to perform acquisition control of each scanning beam plane and configure detector parameters, etc. For example, the scan control system instruction may include signals such as angle coded A-direction pulse signal, angle coded Z-direction pulse signal, belt coded A-direction pulse signal, and belt coded Z-direction pulse signal.

According to an embodiment of the present disclosure, sending the internal control instruction to a plurality of acquisition controllers in step S03 may include: sending the internal control instruction to the plurality of acquisition controllers by the master controller via a plurality of high-speed serial interfaces.

By using this method, all acquisition controllers are not connected to the CAN bus, but achieve instruction interaction with the scan control system through the master controller as a hub, which may reduce the number of CAN nodes in the system and improve the stability and reliability of the data acquisition system.

By way of example, the high-speed serial interface may include a high-speed serial optical fiber interface. The high-speed serial optical fiber interface provides high transmission speed and low latency. By transmitting the internal control instruction to all acquisition controllers through the high-speed serial optical fiber interface, it is possible to achieve synchronous sampling of all beam planes. Therefore, the scan control system does not need to provide the beam plane synchronization signal.

By using this method, the control signals of the scan control system (such as angle coded A-direction pulse signal, angle coded Z-direction pulse signal, belt coded A-direction pulse signal, belt coded Z-direction pulse signal, etc.) are not directly provided to the acquisition controllers. Instead, they are directly connected to the master controller, and the master controller may encode the control signals to generate an internal control instruction, which is then transmitted to the plurality of acquisition controllers, thereby greatly reducing the number of control signals and improving the stability and reliability of the data acquisition system.

According to an embodiment of the present disclosure, parsing the scan control system instruction to generate an internal control instruction in step S02 may include: generating a sampling control signal and a sampling time information according to the scan control system instruction; and encoding the sampling control signal and the sampling time information to generate the internal control instruction.

By way of example, the sampling time information may include information such as angle code A, angle code Z (scan loop number), belt code A, and belt code Z.

By way of example, the acquisition controller may use data information such as angle code A, angle code Z (scan loop number), belt code A, and belt code Z as unique identifiers for corresponding detection data, which endows the detection data with temporal attributes and improves the accuracy of the detection data. For example, angle code A and angle code Z may be used to respectively identify the number of scan loops and the scanning angle in each loop of the scanning imaging system. When a large amount of detection data is generated due to numerous scan loops, angle code A and angle code Z may still accurately identify slice information and scanning information corresponding to each detection data, thus characterizing the temporal attributes of the detection data. Belt code A and belt code Z may be used to respectively identify the number of rotation loops and the rotation angle of a belt pulley. When a large number of objects to be scanned are placed on the conveyor belt, belt code A and belt code Z may be used to accurately identify the position information of each object to be scanned corresponding to the detection data, thus characterizing the temporal attribute of the detection data.

According to an embodiment of the present disclosure, continuing to refer to FIG. 4, the data acquisition method for static CT scanning may further include step S05 to step S07.

In step S05, the acquired data is uploaded by the plurality of detectors to the corresponding acquisition controllers.

In step S06, the acquired data and the sampling time information are packetized by the plurality of acquisition controllers to obtain a plurality of data packets.

In step S07, the plurality of data packets are sent to the master controller by the plurality of acquisition controllers.

FIG. 5 shows a schematic structural diagram of a data packet according to an embodiment of the present disclosure.

By way of example, referring to FIG. 5, a data packet 50 may include beam plane code 501, angle code 502, belt code 503, and detector data 504. For example, the angle code 502 may include angle code A and angle code Z. The belt code 503 may include belt code A and belt code Z. In an embodiment of the present disclosure, the data packet 50 may be a data sequence. For example, the data sequence is formed by encoding the beam plane code 501, the angle code 502, the belt code 503 and the detector data 504. The embodiments of the present disclosure do not impose limitations to the number of bytes of the data sequence and the number of bytes respectively occupied by the beam plane code 501, the angle code 502, the belt code 503, and the detector data 504. For example, the beam plane code 501, the angle code 502, and the belt code 503 may respectively occupy 2 bytes, 4 bytes, and 4 bytes. The number of bytes occupied by the detector data 504 may vary depending on the number of scanning slices.

In an embodiment of the present disclosure, in the data packet 50, the detector data 504 varies in real time with the angle code 502 and the belt code 503, and the detector data 504 corresponds to the angle code 502 and the belt code 503 one to one. For example, when the angle code 502 and the belt code 503 change in real time, the detector data 504 also changes in real time. The detector data 504 may include a plurality of detection data, and the angle code 502 and the belt code 503 may respectively include a plurality of angle data and a plurality of belt data. When an angle data and a belt data are generated, a corresponding detection data is also acquired.

According to an embodiment of the present disclosure, the data acquisition method for static CT scanning may further include: uploading the plurality of data packets to an acquisition server by the master controller.

By way of example, the acquisition server may include a data acquisition server. The data acquisition server may further process the acquired data to obtain slice data. For example, based on the same Z-direction angle code in the plurality of data packets, it is possible to acquire all detection data corresponding to the same slice. Since the plurality of data packets have the same time reference, reordering the detection data corresponding to the same Z-direction angle code in the plurality of data packets allows for accurate generation of the slice data of the slice corresponding to the Z-direction angle code. The slice data is two-dimensional data and represents an image information of the corresponding slice. A three-dimensional reconstruction of the object to be scanned may be performed based on the plurality of slice data, thereby obtaining a three-dimensional image.

By way of example, data transmission between the master controller and the data acquisition server may be performed via a multi-port 10-gigabit Ethernet or a high-speed serial computer expansion bus.

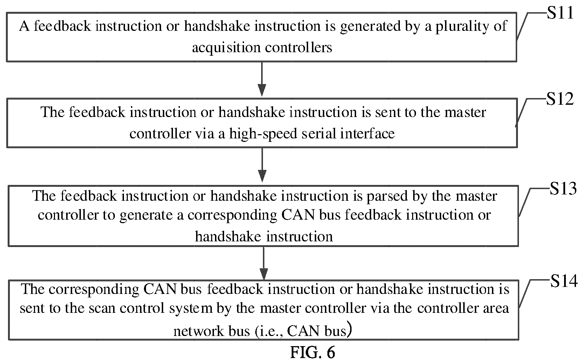

FIG. 6 shows a partial flowchart of a data acquisition method for static CT scanning according to an embodiment of the present disclosure.

According to an embodiment of the present disclosure, referring to FIG. 6, after completing data acquisition, the data acquisition method for static CT scanning further includes step S11 to step S14.

In step S11, a feedback instruction or handshake instruction is generated by a plurality of acquisition controllers.

In step S12, the feedback instruction or handshake instruction is sent to the master controller via a high-speed serial interface.

In step S13, the feedback instruction or handshake instruction is parsed by the master controller to generate a corresponding CAN bus feedback instruction or handshake instruction.

In step S14, the corresponding CAN bus feedback instruction or handshake instruction is sent to the scan control system by the master controller via the CAN bus.

By feeding back relevant instructions to the scan control system, the scan control system may further optimize the control signals according to the feedback instruction, thereby further improving the accuracy of the data acquisition method.

In the embodiments of the present disclosure, by adopting a master-controller-based distributed data acquisition method for static CT, the number of system control signals and the number of CAN nodes may be significantly reduced, and the stability and reliability of the system may be improved.

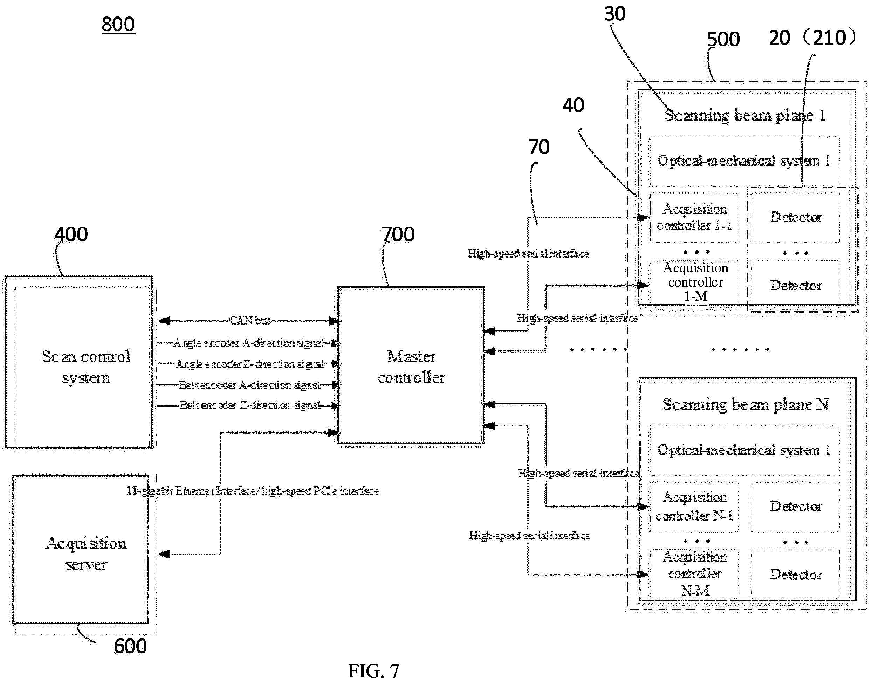

FIG. 7 shows a schematic structural diagram of a data acquisition system for static CT scanning according to an embodiment of the present disclosure.

An embodiment of the present disclosure further provides a data acquisition system 800 for static CT scanning. Referring to FIG. 7, the data acquisition system 800 for static CT scanning may include: a scan control system 400, a master controller 700, a plurality of scanning beam planes 500, and an acquisition server 600.

By way of example, the plurality of scanning beam planes 500 may include a plurality of scanning imaging systems. For example, a scanning beam plane may include a single scanning imaging system.

By way of example, the acquisition server 600 includes a data acquisition server. The acquisition server 600 may receive the acquired data from the plurality of scanning beam planes 500, and process the acquired data to form CT slice data.

By way of example, the scan control system 400 is configured to send a scan control system instruction to the master controller 700. For example, the scan control system instruction may include a CAN bus communication instruction. The scan system control instruction may be used to perform an acquisition control of each scanning beam plane, configure detector parameters, etc. For example, the scan control system instruction may include signals such as angle coded A-direction pulse signal, angle coded Z-direction pulse signal, belt coded A-direction pulse signal, and belt coded Z-direction pulse signal.

By way of example, the master controller 700 is configured to parse the scan control system instruction and generate an internal control instruction. For example, the master controller 700 may parse and decode the CAN bus communication instruction to generate an internal control instruction. For example, the internal control instruction may include a serial communication instruction.

Through such a design, the control signals of the scan control system are not directly provided to the acquisition controller but are directly connected to the master controller, which may reduce the number of CAN nodes and the number of control signal cables, thereby reducing wiring requirements and improving the stability and reliability of the system.

By way of example, a plurality of high-speed serial interfaces 70 are configured to transmit the internal control instruction to the plurality of acquisition controllers 40 respectively.

By transmitting the internal control instruction to all acquisition controllers via a plurality of high-speed serial optical fiber interfaces, it is possible to achieve synchronous sampling of all beam planes, so that the scan control system does not need to provide beam plane synchronization signals, and the number of system control signals may be reduced.

By way of example, at least one scanning imaging system of the plurality of scanning beam planes 500 may include one or more acquisition controllers 40, and the acquisition controller 40 is configured to control the detector 210 to perform data acquisition according to the internal control instruction.

By way of example, a scanning beam plane may include a plurality of acquisition controllers 40 and a plurality of detectors 210. By way of example, a plurality of acquisition controllers 40 and a plurality of detectors 210 in a scanning beam plane may be set in one-to-one correspondence. For example, referring to FIG. 7, the scanning beam plane 1 includes acquisition controller 1-1, ..., acquisition controller 1-M, and a plurality of detectors (e.g., M detectors). The plurality of acquisition controllers 40 may respectively control the plurality of detectors 210 to perform data acquisition. For example, the acquisition controller 1-1 may control a corresponding detector to perform data acquisition, and the acquisition controller 1-M may control another corresponding detector to perform data acquisition.

Through such a design, all acquisition controllers are not connected to the CAN bus but achieve instruction interaction with the scan control system through the master controller as a hub, thereby reducing the number of CAN nodes in the system.

By way of example, the scanning imaging system 800 may further include an optical-mechanical system 30 (also referred to as a multi-target array optical-mechanical system). The optical-mechanical system 30 is configured to emit scanning rays.

By way of example, the scanning imaging system 800 may further include one or more detectors 210. For example, a plurality of detectors 210 may form a detector array 20. The detectors 210 are configured to receive scanning rays that have passed through the object to be scanned and generate detection data based on the received scanning rays.

According to an embodiment of the present disclosure, the acquisition controller 40 is further configured to packetize the acquired data and the sampling time information to obtain a plurality of data packets; and send the plurality of data packets to the master controller 700 via a plurality of high-speed serial interfaces 70.

According to an embodiment of the present disclosure, the scanning imaging system 800 may further include a controller area network bus (i.e., CAN bus). The CAN bus is configured for signal transmission between the scan control system 400 and the master controller 700.

According to an embodiment of the present disclosure, the scanning imaging system 800 may further include an acquisition server 600. The acquisition server 600 may be a data acquisition server. Data transmission between the data acquisition server and the master controller 700 may be performed via a multi-port 10-gigabit Ethernet or a high-speed serial computer expansion bus (referred to as high-speed PCIe).

In the embodiments of the present disclosure, by adopting a master-controller-based distributed data acquisition system for static CT, the number of system control signals and the number of CAN nodes may be significantly reduced, and the stability and reliability of the system may be improved.

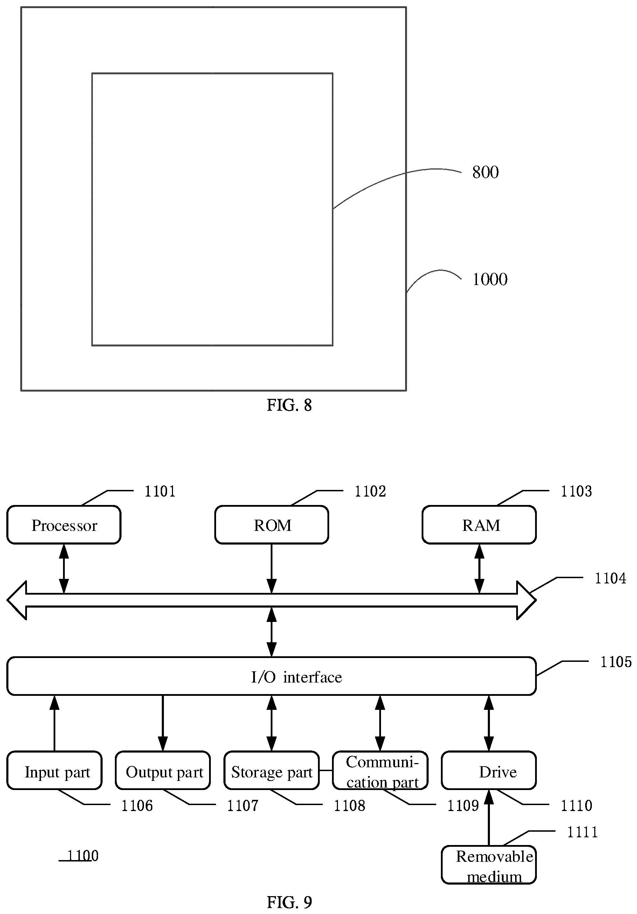

FIG. 8 shows a structural block diagram of a CT device according to an embodiment of the present disclosure.

By way of example, an embodiment of the present disclosure further provides a CT device 1000. Referring to FIG. 8, the CT device 1000 may include the data acquisition system 800 as described above. It should be understood that the CT device has the same beneficial effects as the data acquisition system provided in the foregoing embodiments.

FIG. 9 schematically shows a block diagram of an electronic device suitable for implementing the functions of controlling a CT device and/or the functions of processing data of the CT device according to an embodiment of the present disclosure.

As shown in FIG. 9, an electronic device 1100 according to an embodiment of the present disclosure includes a processor 1101, which may execute various appropriate actions and processing according to the program stored in a read only memory (ROM) 1102 or the program loaded into a random access memory (RAM) 1103 from a storage part 1108. The processor 1101 may include, for example, a general-purpose microprocessor (for example, CPU), an instruction set processor and/or a related chipset and/or a special-purpose microprocessor (for example, an application specific integrated circuit (ASIC)), and the like. The processor 1101 may further include an on-board memory for caching purposes. The processor 1101 may include a single processing unit or multiple processing units for executing different actions of the method flow according to embodiments of the present disclosure.

Various programs and data required for the operation of the electronic device 1100 are stored in the RAM 1103. The processor 1101, the ROM 1102 and the RAM 1103 are connected to each other through a bus 1104. The processor 1101 executes various operations of the method flow according to embodiments of the present disclosure by executing the programs in the ROM 1102 and/or the RAM 1103. It should be noted that the program may also be stored in one or more memories other than the ROM 1102 and the RAM 1103. The processor 1101 may also execute various operations of the method flow according to embodiments of the present disclosure by executing the programs stored in the one or more memories.

According to embodiments of the present disclosure, the electronic device 1100 may further include an input/output (I/O) interface 1105, which is also connected to the bus 1104. The electronic device 1100 may further include one or more of the following components connected to the I/O interface 1105: an input part 1106 including a keyboard, a mouse, etc.; an output part 1107 including a cathode ray tube (CRT), a liquid crystal display (LCD), etc. and a speaker, etc.; a storage part 1108 including a hard disk, etc.; and a communication part 1109 including a network interface card such as a LAN card, a modem, and the like. The communication part 1109 performs communication processing via a network such as the Internet. A drive 1110 is also connected to the I/O interface 1105 as required. A removable medium 1111, such as a magnetic disk, an optical disk, a magneto-optical disk, a semiconductor memory, and the like, is installed on the drive 1110 as required, so that the computer program read therefrom is installed into the storage part 1108 as needed.

Those skilled in the art may understand that the above-described embodiments are all exemplary and may be improved by those skilled in the art. The structures described in the various embodiments may be freely combined without conflict in structure or principle.

After reading the detailed description of the preferred embodiments of the present disclosure, those skilled in the art may clearly understand that various changes and modifications may be made without departing from the scope and spirit of the appended claims, and the present disclosure is not limited to the implementation manners of the exemplary embodiments provided in the specification.

## Claims

1. A data acquisition method for static computed tomography, comprising:
sending (S01) a scan control system instruction to a master controller;
parsing (S02) the scan control system instruction to generate an internal control instruction;
sending (S03) the internal control instruction to a plurality of acquisition controllers; and
controlling (S04) a plurality of detectors to perform data acquisition according to the internal control instruction.

2. The method according to claim 1, wherein the sending a scan control system instruction to a master controller comprises:
sending the scan control system instruction to the master controller by a scan control system via a controller area network bus.

3. The method according to claim 1 or 2, wherein the sending the internal control instruction to a plurality of acquisition controllers comprises:
sending the internal control instruction to the plurality of acquisition controllers by the master controller via a plurality of high-speed serial interfaces.

4. The method according to claim 3, wherein the scan control system instruction comprises an angle coded A-direction pulse signal, an angle coded Z-direction pulse signal, a belt coded A-direction pulse signal, and a belt coded Z-direction pulse signal.

5. The method according to claim 4, wherein the parsing the scan control system instruction to generate an internal control instruction comprises:
generating a sampling control signal and a sampling time information according to the scan control system instruction; and
encoding the sampling control signal and the sampling time information to generate the internal control instruction.

6. The method according to claim 5, wherein the sampling time information comprises angle code A, angle code Z, belt code A, and belt code Z.

7. The method according to claim 5, further comprising:
uploading (S05) acquired data to the corresponding acquisition controllers by the plurality of detectors;
packetizing (S06) the acquired data and the sampling time information by the plurality of acquisition controllers to obtain a plurality of data packets; and
sending (S07) the plurality of data packets to the master controller by the plurality of acquisition controllers.

8. The method according to claim 7, further comprising:
uploading the plurality of data packets to an acquisition server by the master controller, wherein a data transmission between the master controller and the acquisition server is performed via a multi-port 10-gigabit Ethernet or a high-speed serial computer expansion bus.

9. The method according to claim 3, further comprising: after completing data acquisition,
generating (S11) a feedback instruction or handshake instruction by the plurality of acquisition controllers;
sending (S12) the feedback instruction or handshake instruction to the master controller via the high-speed serial interface;
parsing (S13) the feedback instruction or handshake instruction by the master controller to generate a corresponding controller area network bus feedback instruction or handshake instruction; and
sending (S14) the corresponding controller area network bus feedback instruction or handshake instruction to the scan control system by the master controller via the controller area network bus.

10. A data acquisition system for static computed tomography, comprising:
a scan control system configured to send a scan control system instruction to a master controller;
the master controller configured to parse the scan control system instruction to generate an internal control instruction;
a plurality of high-speed serial interfaces configured to transmit the internal control instruction to a plurality of acquisition controllers; and
a plurality of scanning imaging systems, wherein the scanning imaging system comprises one or more acquisition controllers, and the acquisition controller is configured to control a detector to perform data acquisition according to the internal control instruction.

11. The system according to claim 10, wherein the scanning imaging system further comprises:
an optical-mechanical system configured to emit scanning rays; and
one or more detectors configured to receive scanning rays that have passed through an object to be scanned, and generate detection data based on the received scanning rays.

12. The system according to claim 10, wherein the acquisition controller is further configured to:
packetize acquired data and sampling time information to obtain a plurality of data packets; and
send the plurality of data packets to the master controller via the plurality of high-speed serial interfaces.

13. The system according to claim 10, further comprising:
a controller area network bus configured for signal transmission between the scan control system and the master controller.

14. The system according to claim 10, further comprising an acquisition server, wherein data transmission between the acquisition server and the master controller is performed via a multi-port 10-gigabit Ethernet or a high-speed serial computer expansion bus.

15. A CT device, comprising the data acquisition system according to any one of claims 10 to 14.
